(19) Europäisches Patentamt
European Patent Office
Office européen des brevets

(11) EP 0 533 048 B2

(12) NEUE EUROPÄISCHE PATENTSCHRIFT

(45) Veröffentlichungstag und Bekanntmachung des Hinweises auf die Entscheidung über den Einspruch:
**24.03.2004 Patentblatt 2004/13**

(45) Hinweis auf die Patenterteilung:
**15.03.1995 Patentblatt 1995/11**

(21) Anmeldenummer: **92115440.7**

(22) Anmeldetag: **09.09.1992**

(51) Int Cl.[7]: **C07D 471/08**
// C07D211/86,(C07D471/08, 221:00, 209:00)

(54) **Verfahren zur Herstellung von Lactamen**

Process for the preparation of lactams

Procédé de préparation de lactames

(84) Benannte Vertragsstaaten:
**AT BE CH DE DK FR GB IE IT LI LU NL SE**

(30) Priorität: **12.09.1991 CH 269791**

(43) Veröffentlichungstag der Anmeldung:
**24.03.1993 Patentblatt 1993/12**

(73) Patentinhaber: **LONZA AG**
**CH-3945 Gampel/Wallis (CH)**

(72) Erfinder:
- **Griffiths, Gareth, Dr.**
  **Visp (Kanton Wallis) (CH)**
- **Previdoli, Felix, Dr.**
  **Brig (Kanton Wallis) (CH)**

(74) Vertreter:
**Weinhold, Peter, Dr.rer.nat. Dipl.-Chem. et al**
**Winter, Brandl, Fürniss, Hübner,**
**Röss, Kaiser, Polte**
**Partnerschaft**
**Patent- und Rechtsanwaltskanzlei**
**Alois-Steinecker-Strasse 22**
**85354 Freising (DE)**

(56) Entgegenhaltungen:
**DE-A- 1 930 014**

- **JOURNAL OF ORGANIC CHEMISTRY Bd. 43, Nr. 12, 1978, Seiten 2311 - 2320;'synthesis of carbocyclic aminonucleosides'**
- **ORGANIC SYNTHESIS Bd. 57, 1977, Seiten 88 - 92; 'sulfonyl cyanides:methanesulfonyl cyanide'**
- **TETRAHEDRON LETTERS Bd. 39, 1969, Seiten 3351 - 3352; 'a simple synthesis of sulphonyl cyanides'**
- **Journal of Organic Chemistry, 1974, Bd. 39, S. 564-566**
- **Journal of Organic Chemistry, 1993, Bd. 58, S. 6129-6131**

EP 0 533 048 B2

## Beschreibung

Die vorliegende Erfindung betrifft ein Verfahren zur Herstellung von β,γ-ungesättigten δ-Lactamen aus 1,3-Dienen und Chlorcyan.
Die erfindungsgemäss hergestellten Lactame können durch die allgemeine Formel

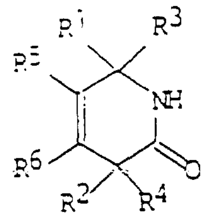

I

wiedergegeben werden, worin $R^1$ und $R^2$ entweder unabhängig voneinander Wasserstoff, Alkyl, Aryl, Alkoxy, Aryloxy, Alkylthio, Arylthio oder Trialkylsilyloxy bedeuten oder zusammen eine Methandiyl- oder Ethandiylgruppe, also eine $-(CH_2)_n$-Brücke mit n = 1 bis 2, bilden,
$R^3$ und $R^4$ unabhängig voneinander Wasserstoff, Alkyl, Aryl, Alkoxy, Aryloxy, Alkylthio, Arylthio oder Trialkylsilyloxy bedeuten
und $R^5$ und $R^6$ entweder unabhängig voneinander Wasserstoff, Alkyl, Aryl, Alkoxy, Aryloxy, Alkylthio, Arylthio oder Trialkylsilyloxy bedeuten oder zusammen mit den beiden durch die Doppelbindung verbundenen Kohlenstoffatomen des Lactamrings einen carbocyclischen oder heterocyclischen Ring mit 5 bis 8 Ringgliedern bilden.

Bei den obigen Bedeutungen Alkyl, Alkoxy und Alkylthio handelt es sich um Reste mit 1 bis 4 Kohlenstoffatomen, beispielsweise Methyl, Ethyl, Propyl, Isopropyl, Butyl, Methoxy, Ethoxy, Propoxy, Isopropoxy, Methylthio oder Ethylthio.

Bei Aryl, Aryloxy und Arylthio handelt es sich um substituierte oder unsubstituierte aromatische Reste mit 6 bis 12 Kohlenstoffatomen, beispielsweise Phenyl, Tolyl, Phenoxy oder Phenylthio.

Trialkylsilyloxy ist zweckmäßig Trimethylsilyloxy oder Triethylsilyloxy.

Besonders bevorzugte Produkte des erfindungsgemäßen Verfahrens sind die, worin entweder $R^3$ und $R^4$ Wasserstoff sind und/oder $R^5$ und $R^6$ unabhängig voneinander Wasserstoff oder $C_1$-$C_4$ Alkyl sind, und/oder $R^1$ und $R^2$ zusammen eine Methandiylgruppe bilden und/oder R ausgewählt ist aus der Gruppe bestehend aus Methyl, Ethyl, Phenyl, p-Tolyl und p-Bromophenyl.

Ein besonders bevorzugtes Produkt des erfindungsgemäßen Verfahrens ist das 2-Azabicyclo[2.2.1]hept-5-en-3-on, das den Fall $R^1$,$R^2$ = $-CH_2-$, $R^3$ = $R^4$ = $R^5$ = $R^6$ = H der allgemeinen Formel I darstellt.

2-Azabicyclo[2.2.1]hept-5-en-3-on ist ein bicyclisches Lactam, das als Ausgangsmaterial für die Synthese von carbocyclischen Nucleosidanalogen geeignet ist (S.Daluge und R.Vince. J.Org.Chem. 43, S.2311 (1978)). Solche Nucleosidanaloge sind wegen ihrer antiviralen und chemotherapeutischen Eigenschaften als potentielle Antitumormittel von Interesse. Eine bekannte Synthese von 2-Azabicyclo[2.2.1]hept-5-en-3-on beruht auf der Diels-Alder-Reaktion von Cyclopentadien mit p-Toluolsulfonylcyanid. Hierbei entsteht zunächst ein Tosyl-azanorbornadien, das durch saure oder alkalische Hydrolyse in die Zielverbindung übergeführt wird (J.C.Jagt und A.M.van Larsen, J.Org.Chem. 39, S. 564 (1974); S.Daluge und R.Vince, loc. cit.).
Nachteile dieses Verfahrens sind die Explosionsneigung von p-Toluolsulfonylcyanid und das ungünstige Mengenverhältnis des Produkts zum Abfallprodukt p-Tolylsulfinyl-p-tolylsulfon. Ausserdem wird das Cyclopentadien in grossem Ueberschuss eingesetzt, der vor der Hydrolyse abdestilliert werden muss.

Aufgabe der vorliegenden Erfindung war, ein Verfahren zur Herstellung von 2-Azabicyclo[2.2.1]hept-5-en-3-on und anderen durch Diels-Alder-Reaktion von 1,3-Dienen mit Sulfonylcyaniden herstellbaren β,γ-ungesättigten δ-Lactamen bereitzustellen, welches von leicht zugänglichen Ausgangsmaterialien ausgeht und keine grossen Mengen von Neben- bzw. Abfallprodukten liefert.

Erfindungsgemäss wird die Aufgabe durch das Verfahren nach Patentanspruch 1 gelöst.

Es wurde überraschend gefunden, dass es nicht nur möglich ist, das Sulfonylcyanid in situ aus dem entsprechenden Sulfonylchlorid, einer Base, einem Reduktionsmittel und Chlorcyan herzustellen und mit dem 1,3-Dien zu cyclisieren, sondern dass das nach der Cycloaddition bei der Abspaltung der Alkyl- bzw. Arylsulfonylgruppe entstehende Alkan- bzw. Arensuifinat mit weiterem Chlorcyan unter den gleichen Reaktionsbedingungen wieder zum Sulfonylcyanid regeneriert und erneut zur Cycloaddition verwendet werden kann. Damit ist es möglich, mit einer katalytischen Menge an Sulfonylchlorid bzw. Sulfonylcyanid auszukommen und entsprechend wenig Abfall zu produzieren.
Der dem erfindungsgemässen Verfahren zugrundeliegende Kreisprozess lässt sich durch folgendes Schema veran-

schaulichen:

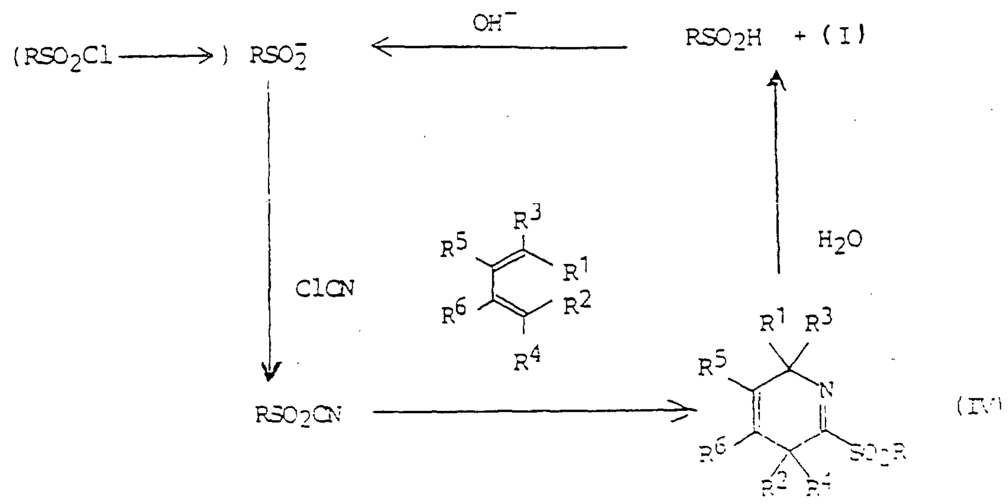


Verbraucht werden also nur das Dien, Chlorcyan und Hydroxidionen entsprechend der idealisierten Bruttogleichung:

$$\text{Dien} + \text{ClCN} + \text{OH}^- \rightarrow \text{Lactam (I)} + \text{Cl}^-.$$

Der gesamte Reaktionscyclus einschliesslich der Reduktion des Sulfonylchlorids zum für die Bildung der Anfangsmenge Sulfonylcyanid benötigten Sulfinat kann als Eintopfreaktion durchgeführt werden. Es liegt selbstverständlich auch im Rahmen der Erfindung, das Sulfinat oder das Sulfonylcyanid als solches als Startverbindung zu wählen und den Reaktionscyclus an der entsprechenden Stelle zu beginnen.

Als Diene für das erfindungsgemässe Verfahren eignen sich insbesondere reaktive elektronenreiche Diene, wie beispielsweise 1,3-Cyclopentadien, 1,3-Cyclohexadien, Isopren und 1,3-Butadien oder Diene mit elektronenliefernden Substituenten, wie beispielsweise den oben näher definierten Alkylgruppen, Arylgruppen, Alkoxygruppen, Aryloxygruppen, Alkylthiogruppen, Arylthiogruppen oder Trialkylsilyloxygruppen.

Wenn das Dien unter den Reaktionsbedingungen genügend stabil ist, wie beispielsweise 1,3-Cyclopentadien, kann die gesamte Dienmenge vorgelegt werden, andernfalls kann es entsprechend dem Reaktionsfortschritt zudosiert werden.

Als Sulfonylkomponenten eignen sich Alkan- oder Arensulfonsäurehalogenide und -pseudohalogenide, insbesondere Alkan- oder Arensulfonsäurechloride oder -cyanide, oder Alkan- oder Arensulfinsäuren bzw. deren Salze. Vorzugsweise werden kommerziell erhältliche wie Methansulfonyl- (Mesyl-), Ethansulfonyl-, Benzolsulfonyl-, p-Toluolsulfonyl- (Tosyl-) oder p-Brombenzolsulfonyl- (Brosyl-) -chlorid bzw. -cyanid eingesetzt.

Die Sulfonylkomponente wird in einer Menge von weniger als 0,5 mol, bezogen auf 1 Mol des Diens, eingesetzt. Besonders bevorzugt sind Mengen von 0,2 Mol und weniger.

Die Cycloaddition wird in neutralem bis mässig saurem Milieu durchgeführt, d.h. bei einem pH von 2 bis 7, vorzugsweise in schwach saurem Milieu bei einem pH von 4 bis 6. Unter diesen Bedingungen laufen sowohl die Hydrolyse des Diels-Alder Addukts (IV) zum Lactam (I) als auch die Rückbildung des Sulfonylcyanids aus dem Sulfinat ab, so dass der katalytische Cyclus geschlossen ist.

Da bei der Reaktion Chlorwasserstoff entsteht, muss der pH durch Zugabe einer Base konstant gehalten werden, wenn nicht ein gepuffertes System eingesetzt wird.
Als Base eignen sich sowohl organische Basen, wie beispielsweise quartäre Ammoniumhydroxide, als auch anorganische Basen, wie beispielsweise Alkali- oder Erdalkalihydroxide. Vorzugsweise wird als Base ein Alkalihyaroxid eingesetzt, besonders bevorzugt ist Natriumhydroxid. Die Basenzugabe erfolgt vorzugsweise mittels einer geeigneten Regelvorrichtung, bestehend aus einem pH-Messgerät und einer von diesem gesteuerten Dosiervorrichtung.

Als Lösungsmittel wird zweckmässig ein Lösungsmittel eingesetzt, das gegen die im Reaktionscyclus auftretenden Verbindungen entweder inert ist oder höchstens langsam mit ihnen reagiert. Hierzu zählen beispielsweise Wasser, Acetonitril, Tetrahydrofuran, Dioxan, Dichlormethan, Toluol oder Mischungen oder zweiphasige Systeme aus diesen Lösungsmitteln. Vorzugsweise wird die Cycloaddition in einem wässrigen Lösungsmittelgemisch oder in einem intensiv gerührten zweiphasigen System aus Wasser und einem nicht mit Wasser mischbaren organischen Lösungsmittel durchgeführt. Besonders bevorzugt sind Tetrahydrofuran und das System Wasser/Dichlormethan.

Die Cycloaddition wird vorzugsweise bei einer Temperatur von -5 bis 25°C durchgeführt, besonders bevorzugt bei + 5 bis 20 °C.

Die Aufarbeitung des Reaktionsgemischs kann in an sich bekannter Weise erfolgen, beispielsweise durch Extraktion mit einem Lösungsmittel geringer Polarität.

Die nachfolgenden Beispiele verdeutlichen die Durchführung des erfindungsgemässen Verfahrens.

Beispiel 1

2-Azabicyclo[2.2.1]hept-5-en-3-on

Zu einer Lösung von 21,4 g (0,17 mol) Natriumsulfit und 28,6 g (0,34 mol) Natriumhydrogencarbonat in 330 ml Wasser wurden bei 18-20°C 19,5 g (0,17 mol) Methansulfonylchlorid innerhalb von 30 min zugetropft. Das Gemisch wurde über Nacht stehengelassen und dann auf 15°C abgekühlt. Zu der so hergestellten Natriummethansulfinatlösung wurden 88,1 g (1,33 mol) frisch destilliertes Cyclopentadien und 83 ml Dichlormethan gegeben und bei 15°C innerhalb von 5 h unter Rühren 116.9 g (1,90 mol) Chlorcyan eingeleitet.
Mittels einer Regelvorrichtung, bestehend aus einem pH-Meter und einer impulsgesteuerten Dosiervorrichtung, wurde während der Einleitung und danach für weitere 1,75 h der pH durch Zugabe von insgesamt ca. 224 g 30%iger Natronlauge konstant bei 5 gehalten.
Anschliessend wurde der pH durch Zugabe von 10,4 g 30%iger Natronlauge auf 8 eingestellt und das Reaktionsgemisch viermal mit je 290 ml Dichlormethan extrahiert. Die vereinigten organischen Phasen wurden über Magnesiumsulfat getrocknet, filtriert und im Vakuum eingeengt. Der Rückstand wurde im Hochvakuum getrocknet.
Ausbeute: 103,0 g oranges Rohprodukt, Gehalt (HPLC) 95,5%.

Beispiel 2

2-Azabicyclo[2.2.1]hept-5-en-3-on

In 300 ml Wasser wurden 45,9 g (0,25 mol) Natrium-4-toluolsulfinat (Hydrat) gelöst und auf 0°C abgekühlt. Zu dieser Lösung wurden 82,6 g (1,25 mol) Cyclopentadien (frisch destilliert), 100 ml Tetrahydrofuran und 15,0 g (0,25 mol) Essigsäure gegeben und das Gemisch auf -3 bis + 1°C abgekühlt. Bei dieser Temperatur wurden 78,0 g (1,27 mol) Chlorcyan innerhalb von 80 min eingeleitet und das Gemisch anschliessend bei 10°C gerührt.
Analog zu Beispiel 1 wurde der pH durch Zugabe von insgesamt ca. 120 g 30%iger Natronlauge über einen Zeitraum von 6 h bei 4 gehalten. Anschliessend wurde der pH durch Zugabe von weiteren 69 g 30%iger Natronlauge auf 8 erhöht.
Das Gemisch wurde mit 300 ml Dichlormethan und 100 ml Wasser versetzt, die Phasen getrennt und die wässrige Phase zweimal mit je 150 ml Dichlormethan extrahiert.
Die vereinigten organischen Phasen wurden über Magnesiumsulfat getrocknet und filtriert. Das Lösungsmittel wurde im Vakuum abdestilliert und der Rückstand im Hochvakuum getrocknet.

Ausbeute: 80.3 g gelbbraunes Rohprodukt,
Gehalt (HPLC) 78,7%.

## Patentansprüche

1. Verfahren zur Herstellung von Lactamen der allgemeinen Formel

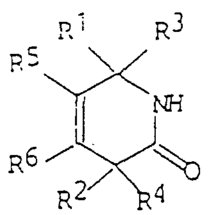

I

worin $R^1$ und $R^2$ entweder unabhängig voneinander Wasserstoff, Alkyl, Aryl, Alkoxy, Aryloxy, Alkylthio, Arylthio

oder Trialkylsilyloxy bedeuten oder zusammen eine Methandiyl- oder Ethandiylgruppe bilden,
$R^3$ und $R^4$ unabhängig voneinander Wasserstoff, Alkyl, Aryl, Alkoxy, Aryloxy, Alkylthio, Arylthio oder Trialkylsilyloxy bedeuten
und $R^5$ und $R^6$ entweder unabhängig voneinander Wasserstoff, Alkyl, Aryl, Alkoxy, Aryloxy, Alkylthio, Arylthio oder Trialkylsilyloxy bedeuten oder zusammen mit den beiden durch die Doppelbindung verbundenen Kohlenstoffatomen des Lactamrings einen carbocyclischen oder heterocyclischen Ring mit 5 bis 8 Ringgliedern bilden, wobei die oben genannten Alkyl-, Alkoxy- und Alkylthio-Gruppen solche mit 1 bis 4 Kohlenstoffatomen sind und die Aryl- Aryloxy- und Arylthio-Gruppen substituierte oder unsubstituierte Reste mit 6 bis 12 Kohlenstoffatomen sind,
**dadurch gekennzeichnet, daß** ein 1,3-Dien der allgemeinen Formel

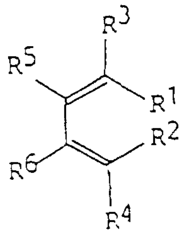

II

worin $R^1$ bis $R^6$ die oben genannten Bedeutungen haben, mit Chlorcyan in Gegenwart von Wasser und einer Sulfinsäure der allgemeinen Formel

$$R\text{-}SO_2H \qquad III$$

worin R eine $C_1$-$C_6$-Alkylgruppe oder eine gegebenenfalls substituierte Phenylgruppe ist, und/oder eines Salzes davon bei einem pH von 2 bis 7 einer Cycloaddition und anschließend einer Hydrolyse unterzogen wird, wobei die Sulfinsäure und/oder das Sulfinat in einer Menge von weniger als 0,5 mol, bezogen auf 1 mol des 1,3-Diens, eingesetzt werden.

**2.** Verfahren nach Patentanspruch 1, **dadurch gekennzeichnet, dass** $R^3$ und $R^4$ Wasserstoff sind.

**3.** Verfahren nach Patentanspruch 1 oder 2, **dadurch gekennzeichnet, dass** $R^5$ und $R^6$ unabhängig voneinander Wasserstoff oder $C_1$-$C_4$-Alkyl sind.

**4.** Verfahren nach einem der Patentansprüche 1 bis 3, **dadurch gekennzeichnet, dass** $R^1$ und $R^2$ zusammen eine Methandiylgruppe bilden.

**5.** Verfahren nach einem der Patentansprüche 1 bis 4, **dadurch gekennzeichnet, dass** R ausgewählt ist aus der Gruppe bestehend aus Methyl, Ethyl, Phenyl, p-Tolyl und p-Bromphenyl.

**6.** Verfahren nach einem der Patentansprüche 1 bis 5, **dadurch gekennzeichnet, dass** die Cycloaddition und die Hydrolyse bei einem pH von 4 bis 6 durchgeführt werden.

**7.** Verfahren nach einem der Patentansprüche 1 bis 6, **dadurch gekennzeichnet, dass** der pH während der Cycloaddition und der Hydrolyse durch Zugabe eines Alkalihydroxids konstant gehalten wird.

**8.** Verfahren nach einem der Patentansprüche 1 bis 7, **dadurch gekennzeichnet, dass** die Cycloaddition und die Hydrolyse in einem Lösungsmittel aus der Gruppe bestehend aus Wasser, Acetonitril, Tetrahydrofuran, Dioxan, Dichlormethan, Toluol und Gemischen dieser Lösungsmittel durchgeführt werden.

**9.** Verfahren nach einem der Patentansprüche 1 bis 8, **dadurch gekennzeichnet, dass** die Cycloaddition und die Hydrolyse bei einer Temperatur von -5 bis +25°C durchgeführt werden.

## Claims

**1.** Process for the preparation of lactams of the general formula

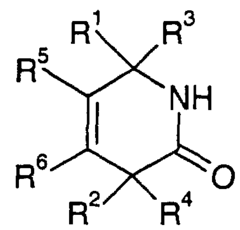

I

wherein $R^1$ and $R^2$, either independently of each other are hydrogen, alkyl, aryl, alkoxy, aryloxy, alkylthio, arylthio, or trialkyl silyloxy, or together are a methanediyl or ethanediyl group,
$R^3$ and $R^4$ independently are hydrogen, alkyl, aryl, alkoxy, aryloxy, alkylthio, arylthio, or trialkyl silyloxy, and
$R^5$ and $R^6$, either independently of each other are hydrogen, alkyl, aryl, alkoxy, aryloxy, alkylthio, arylthio, or trialkyl silyloxy, or together with the two carbon atoms of the lactam ring linked by the double bond constitute a carbocyclic or heterocyclic ring having 5 to 8 ring members, the above mentioned alkyl, alkoxy, and alkylthio groups having 1 to 4 carbon atoms, and the aryl, aryloxy, and arylthio groups being substituted or unsubstitued residues having 6 to 12 carbon atoms,
**characterised in that** a 1,3-diene of the general formula

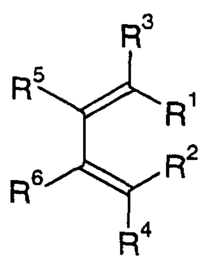

II

wherein $R^1$ to $R^6$ have the above meanings, at a pH of from 2 to 7, is subjected to a cycloaddition reaction with chlorocyan in the presence of water and a sulfinic acid of the general formula

$$R\text{-}SO_2H \qquad III$$

wherein R is a $C_1$-$C_6$ alkyl group or an, optionally substituted, phenyl group, and/or a salt thereof, followed by hydrolysis, the sulfinic acid and/or the sulfinate being employed in an amount of less than 0,5 moles, based on 1 mole of the 1,3-diene.

**2.** Process according to claim 1, **characterised in that** $R^3$ and $R^4$ are hydrogen.

**3.** Process according to claim 1 or 2, **characterised in that** $R^5$ and $R^6$ independently of each other are hydrogen or $C_1$-$C_4$-alkyl.

**4.** Process according to one of claims 1 to 3, **characterised in that** $R^1$ and $R^2$ together constitute a methanediyl group.

**5.** Process according to one of claims 1 to 4, **characterised in that** R is selected from the group consisting of methyl, ethyl, phenyl, p-tolyl, and p-bromophenyl.

**6.** Process according to one of claims 1 to 5, **characterised in that** the cycloaddition reaction and the hydrolysis are

carried out at a pH of from 4 to 6.

7. Process according to one of claims 1 to 6, **characterised in that** during the cycloaddition reaction and the hydrolysis the pH is kept constant by adding an alkali hydroxide.

8. Process according to one of claims 1 to 7, **characterised in that** the cycloaddition reaction and the hydrolysis are carried out in a solvent from the group consisting of water, acetonitrile, tetrahydrofurane, dioxane, dichloromethane, toluene, and mixtures of said solvents.

9. Process according to one of claims 1 to 8, **characterised in that** the cycloaddition reaction and the hydrolysis are carried out at a temperature of from -5 to +25 °C.

**Revendications**

1. Procédé de préparation de lactames de formule générale

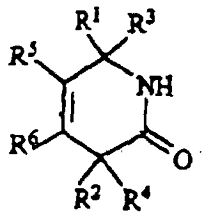

(I)

dans laquelle $R^1$ et $R^2$, soit représentent indépendamment l'un de l'autre un hydrogène, un alkyle, un aryle, un alcoxy, un aryloxy, un alkylthio, un arylthio ou un trialkylsilyloxy, soit forment ensemble un groupe méthanediyle ou éthanediyle,

R3 et R4 représentent indépendamment l'un de l'autre un hydrogène, un alkyle, un aryle, un alcoxy, un aryloxy, un alkylthio, un arylthio, ou un trialkylsilyloxy,

et R5 et R6 soit représentent indépendamment l'un de l'autre un hydrogène, un alkyle, un aryle, un alcoxy, un aryloxy, un alkylthio, un arylthio, ou un trialkylsilyloxy, soit forment ensemble avec les deux atomes de carbone du cycle lactame reliés par la double liaison, un cycle carbocyclique ou hétérocyclique de 5 à 8 chaînons, les groupes alkyle, alcoxy et alkylthio précités étant des groupes de 1 à 4 atomes de carbone et les groupes aryle, aryloxy et arylthio étant des groupements de 6 à 12 atomes de carbone substitués ou non substitués,

**caractérisé en ce que** l'on soumet un 1,3-diène de formule générale

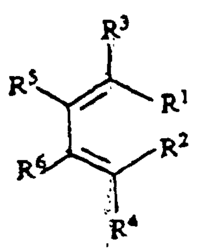

(II)

dans laquelle $R^1$ à $R^6$ ont les significations données ci-dessus, avec du chlorure de cyanogène en présence d'eau et d'un acide sulfinique de formule générale

$$R\text{-}SO_2H \qquad (III)$$

dans laquelle R est un groupe alkyle en $C_1$-$C_6$ ou un groupe phényle éventuellement substitué, et/ou d'un de ses

sels à un pH de 2 à 7 à une cycloaddition puis à une hydrolyse, l'acide sulfinique et/ou le sulfinate étant utilisé en une quantité inférieure à 0,5 mole pour 1 mole du 1,3-diène.

**2.** Procédé selon la revendication 1, **caractérisé en ce que** $R^3$ et $R^4$ sont des hydrogènes.

**3.** Procédé selon la revendication 1 ou 2, **caractérisé en ce que** $R^5$ et $R^6$ sont indépendamment l'un de l'autre un hydrogène ou un alkyle en $C_1$-$C_4$.

**4.** Procédé selon la revendication 1 à 3, **caractérisé en ce que** $R^1$ et $R^2$ forment ensemble un groupe méthanediyle.

**5.** Procédé selon l'une des revendications 1 à 4, **caractérisé en ce que** R est choisi dans le groupe constitué par méthyle, éthyle, phényle, p-tolyle et p-bromophényle.

**6.** Procédé selon l'une des revendications 1 à 5, **caractérisé en ce que** la cycloaddition et l'hydrolyse s'effectuent à un pH de 4 à 6.

**7.** Procédé selon l'une des revendications 1 à 6, **caractérisé en ce que**, pendant la cycloaddition et l'hydrolyse le pH est maintenu à une valeur constante par ajout d'un hydroxyde alcalin.

**8.** Procédé selon l'une des revendications 1 à 7, **caractérisé en ce que** la cycloaddition et l'hydrolyse s'effectuent dans un solvant du groupe constitué par l'eau, l'acétonitrile, le tétrahydrofurane, le dioxanne, le dichlorométhane, le toluène et des mélanges de des solvants.

**9.** Procédé selon l'une des revendications 1 à 8, **caractérisé en ce que** la cycloaddition et l'hydrolyse s'effectuent à une température de -5 à +25°C.